(19) Europäisches Patentamt / European Patent Office / Office européen des brevets

(11) **EP 4 535 160 A1**

(12) **EUROPEAN PATENT APPLICATION**

(43) Date of publication:
09.04.2025 Bulletin 2025/15

(21) Application number: 23202202.0

(22) Date of filing: 06.10.2023

(51) International Patent Classification (IPC):
**G06F 3/16** (2006.01)    **G06N 20/00** (2019.01)
**A61B 5/00** (2006.01)

(52) Cooperative Patent Classification (CPC):
**A61B 5/7267; A61B 5/055; G06N 20/00;**
**G06F 3/167**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Technische Universität München 80333 München (DE)**

(72) Inventor: **The designation of the inventor has not yet been filed**

(74) Representative: **Lucke, Andreas**
**Boehmert & Boehmert**
**Anwaltspartnerschaft mbB**
**Pettenkoferstrasse 22**
**80336 München (DE)**

(54) **DATA-TO-SOUND INTERACTIVE FEEDBACK**

(57)    A method (100) for generating a sound output based on an interaction with a data set, wherein the data set comprises a plurality of data points, each data point storing one or more data features. The method comprises obtaining (102) an interaction with at least a part of the data points of the data set and using (104) a sound model to generate a sound output based on said interaction. The sound model maps at least one of the one or more data features to one or more acoustic properties of the sound output as a function of the interaction. The data features can be one of a spatial feature, a time feature, a physical property, and a data label. The acoustic properties can be one of pitch, pulsing frequency, duty cycle, loudness, and tone colour. The invention further refers to uses of such method in a method of validating labels assigned to ground truth input data and in a method of training a machine learning algorithm, as well as to a system implementing the method for generating a sound output based on an interaction with a data set.

Fig. 1

**Description**

FIELD OF THE INVENTION

The present invention is in the field of data analysis and refers, in particular, to a data-to-sound interactive feedback approach. This approach is implemented by a method for generating a sound output from a user-interaction with a multi-dimensional data set as well as by a related computer program and a related system. The approach may also be used for training a machine learning algorithm and for validating labels assigned to ground truth input data.

BACKGROUND OF THE INVENTION

Data sets are becoming of increasing importance in all types of industries and fields of technology. The advent of data mining techniques and artificial intelligence turn data sets into very powerful tools with implications and applications yet to be fully exploited.

The interpretation and analysis of data sets by human users is typically limited to visual perception, be it by direct visual inspection of the data or by visualization of the data on graphical representation means, such as screens or the like. This imposes upon users the limitation of not being able to use visual perception for other purposes while analyzing said (visualized) data.

In some fields, such as the medical field, this is a major limitation. For example, a surgeon may have to choose, during a surgical treatment, between concentrating their visual perception on data representation and/or data interpretation means, such as a screen showing a scanner or sensor result, or on the body of the patient receiving the surgical treatment.

Thus, there is room for improvement in the field of data analysis.

SUMMARY OF THE INVENTION

The present invention refers to a technique for exploring a data set based on acoustic perception, rather than on visual perception. The invention is based on a data-to-sound interactive feedback approach aiming at overcoming the disadvantages of the prior art mentioned above. This is achieved by a method for generating a sound output based on an interaction with a data set according to claim 1, by a related computer program according to claim 14 and by a related system according to claim 15. Specific uses and applications of the invention are defined in claims 12 and 13.

The invention aims at allowing multisensorial exploration and analysis of a data set, such as of a biomedical image, in particular a 2D image or a 3D image, for example of a CT image, an MRI image or the like. However, the invention may allow exploring and analyzing other types of data sets and/or images, such as data sets obtained via data mining from any industrial or physical system.

A first aspect of the invention refers to a method for generating a sound output from an interaction with a data set. The method may be computer-implemented. The data set comprises a plurality of data points, each data point storing one or more data features. Data points may be understood herein as data entries. Each data point or data entry may correspond to a vector or a matrix comprising a plurality of values, each of the values possibly corresponding to one of the one or more data features. Thus, the data points, and hence the dataset, may be multidimensional and may correspond to a multidimensional grid of data points. The data points may be obtained by one or more sensor devices, for example by one or more sensor devices of a medical imaging device.

For example, for a dataset corresponding to a 3D image of a biological tissue representingone or more physical properties of the tissue, such as density, possibly obtained through a medical imaging technology such as a CT scan, the different data points of the dataset may correspond to different voxels of the image and to a corresponding value, e.g., a density value, such that each data point may comprise three values corresponding to 3 spatial features representing the position in space of the corresponding voxel in one of three space directions, respectively, and a fourth value corresponding in this exemplary case to density, i.e. with each data point P having a vector of four values $\{X, Y, Z, D\}$, X being a position in an X direction, Y being a position in a Y direction, Z being a position in a Z direction and D being a density value. Notably, this is just an explanatory example and in other examples, each data point may have a number of values different to four and not necessarily corresponding to three directions in space and a density value.

The data set may correspond to one or more biomedical images, preferably one or more of a CT image, an MRI image, a PET image, a SPECT image and an OCT image. The data set may correspond to signals resulted from an interaction between imaging waves, in particular electromagnetic waves and/or acoustic waves, and tissue being observed. For example, if the data set corresponds to an interaction between ultrasound imaging waves and a tissue, the data set may comprises radio frequency data.

The data set may also simultaneously correspond to more than one image of one physical system. For example, the data points or voxels of the data set may comprise values corresponding to physical properties extracted from a CT scan of a tissue and may further comprise values corresponding to physical properties extracted from an MRI image of the same tissue, thereby allowing for a multimodal analysis combining information obtained from different imaging techniques.

The sound output may be or may correspond to an audible signal, i.e., to an acoustic signal perceivable by a person. However, the sound output may also correspond to an electronic audio signal encoding a corresponding audible signal, i.e., an electronic signal configured for being provided to some piece of electronic equipment, for example an amplifier and/or a loudspeaker, and for triggering the generation of a corresponding audible signal. The previously mentioned electronic audio signal may be an analogue signal or a digital signal.

The method according to the first aspect of the invention comprises obtaining an interaction with at least a part of the data points of the dataset. The interaction may hence not affect the whole extent of the data set and may instead affect a subset of the data points, for example a given region, group or category of the data points.

"Interaction" may refer herein to any kind of external input received by the dataset and/or by a physical system being represented by the dataset. The interaction may be a virtual or simulated interaction, for example a virtual user-interaction with the data set or with a physical system represented by the data set. For instance, the interaction may correspond to a user navigating a graphical representation of the dataset using an input device such as a keyboard, a joystick, a mouse or the like. Notably, an interaction may also be virtually generated or simulated according to some input parameters without any need to actually generate any physical interaction or movement. For example, it may be possible to simulate ultrasound waves acting upon the physical system. The interaction may however also be a real interaction, for example a direct interaction upon the physical system represented by the data set, for example in the form of radiation or mechanical forces interacting with the physical system or mechanical interaction with a surgical instrument operated by a surgeon. Further examples of the interaction may hence be electromagnetic radiation or ultrasound waves interacting upon the body of the patient a medical image of which is represented by the dataset.

The method further comprises using a sound model to generate a sound output based on said interaction, i.e., as a function of the interaction received by the at least a part of the data points receiving the interaction. The sound model maps at least one of the one or more data features to one or more acoustic properties of the sound output as a function of the interaction. This implies that the sound model may generate a different sound output for different interactions with the same data points of the same data set. In other words, the characteristics of the interaction may determine, along with the one or more data features of the at least a part of the data points affected by the interaction, the sound output generated by the method of the invention.

At least one of the one or more data features corresponds to one of a spatial feature, a time feature, a physical property, and a data label. Examples of a spatial feature may be features representing position, orientation, size and/or shape in physical space, in particular in 3D or 2D space. Examples of a time feature may be a timestamp or a time duration of a physical event represented by the corresponding data point. Examples of a physical property may be any of length, weight, temperature, intensity, density, or any quantifiable physical property. Examples of a data label may be a category of segmentation class or classifier, for example "tumorous tissue" vs "non-tumorous tissue".

As a further example, for a dataset corresponding to a time-recordal of a 3D image of a biological tissue representing tissue density, possibly obtained through a medical imaging technology such as an MRI scan, the different data points of the dataset may correspond to different voxels of the image at a respective time, to a corresponding density value, and to a corresponding category label indicating a type of tissue, such that each data point may comprise six values corresponding to 3 spatial features representing the position in space of the corresponding voxel in one of three space directions, respectively, a corresponding timestamp, a value corresponding to density, and a category label indicating the type of tissue, i.e., with each data point $P$ having a vector of six values $\{X, Y, Z, T, D, C\}$, $X$ being a position in an $X$ direction, $Y$ being a position in a $Y$ direction, $Z$ being a position in a $Z$ direction, $T$ being a timestamp or time value, $D$ being a density value and $C$ being a category label, such as "lung tissue" or "heart tissue" or the like. Notably, this is just an explanatory example and in other examples, each data point may have a number of values different to six and not necessarily corresponding to three directions in space, a timestamp, a density value and a data label.

At least one of the one or more acoustic properties of the sound output corresponds to one of pitch, pulsing frequency, duty cycle, loudness, and tone colour. "Pitch" refers herein to a sound frequency and is measurable in Hz. "Pulsing frequency" may refer herein to an intermittency frequency of the acoustic output. The acoustic output, when being audible sound or used for producing audible sound, can be perceived by the human ear as a sequence of sounds regularly repeating with periods corresponding to (the inverse of) the pulsing frequency. For example, a pulsing frequency of 1 Hz may refer to the fact that the acoustic output may be an intermittent signal alternating active periods of non-zero amplitude with inactive periods of zero amplitude periodically repeating in intervals of 1s. The alternating active periods of non-zero amplitude and the inactive periods of zero amplitude may have the same duration (for example 0,5 s and 0,5 s, respectively, for a pulsing frequency of 1 $s^{-1}$) or different durations (for example 0,8 s and 0,2 s, respectively, for a pulsing frequency of 1 $s^{-1}$). "Duty cycle" may refer herein to a fraction of one period of an intermittent (pulsing) acoustic output over which the signal is active, i.e., has a non-zero amplitude. A duty cycle of 0,5 corresponds to a pulsing signal in which the alternating active periods of non-zero amplitude have the same duration as the inactive periods of zero amplitude (for example 0,5 s and 0,5 s, respectively, for a pulsing frequency of 1 Hz). A duty cycle of 1 corresponds to a continuous non-intermittent signal. Notably, the pitch, the pulsing frequency and/or the duty cycle of an auditory signal can be independently and/or simultaneously perceived by the human ear. For a given auditory signal, the human ear can for example identify when a pulsing frequency or a duty cycle of the auditory signal is varying (i.e., is pulsing slower or faster) and can further independently identify, possibly at the same time, whether a pitch of the signal is varying (i.e., is becoming higher or lower). "Tone colour", also named "timbre", may refer herein to an acoustic property that distinguishes different types of sound production, such as choir voices, sound resulting from mechanical action with different materials, or different musical instruments.

The sound model may map each of the one or more data features to one corresponding acoustic property of the sound output, wherein the acoustic properties may preferably be different from each other. In some embodiments, at least two of the one or more data features, for example spatial position and density, may be mapped by the sound model to two different corresponding acoustic properties of the sound output, for example to pitch and pulsing frequency, respectively.

The mapping between the data features and the acoustic properties determined by the sound model may be a one-to-one map, but this is not necessarily the case. The mapping may also be a many-to-one map, for example were a complete subset of data points (voxels) within a medical image of a tissue and/or values of physical properties thereof are all associated to one given segmentation class, such as "vascular tissue".

Notably, the mapping implemented by the sound model is between the one or more data features and the one or more acoustic properties, i.e., at a feature level, not necessarily between specific data points and the one or more acoustic properties. In particular, values of the data features corresponding to different data points may be used for determining corresponding values of the acoustic properties. Thus, the sound output generated by the sound model may represent non-localized properties of a physical system represented by the data set involving the interaction of more than one data set, for example the interaction of one given voxel of a medical image with other surrounding voxels.

In an example in which the dataset represents a medical image of a tissue comprising tumorous tissue and non-tumorous tissue, the sound model may for example map the position along a given direction to pitch and the segmentation class "tumorous tissue" vs "non-tumorous tissue" to duty cycle, such that a continuous sound is heard while the interaction takes place, along said given direction, with data points having the segmentation class "tumorous tissue" and a pulsing sound is heard while the interaction takes place, along said given direction, with data points having the segmentation class "non-tumorous tissue".

According to the invention, the sound output is generated having said one or more acoustic properties to which the at least one of the one or more data features are mapped by the sound model. Thus, the method of the first aspect of the invention results in the generation of a sound output that encodes the data features of the dataset. Accordingly, the method of the invention allows providing audible information about the data features of the data set. This audible information can convey knowledge about the spatial structure, the time evolution and/or the physical condition of a physical system represented by the data set. Since the information is suitable for being perceived via audition, the perception of such information can easily be combined with the simultaneous perception of further information, in particular via visual perception. This allows enhancing the perception of information by the receptor of the information.

For example, during a medical procedure in which a surgeon may be using an interaction tool, such as a surgical device, the method of the invention may be used to navigate a dataset corresponding to a real-time image of the body of the patient undergoing surgery and a surgeon can simultaneously perceive information about the body of the patient in the form of audible feedback provided by the method of the invention and, additionally, in the form of visual feedback by directly staring at the body of the patient or at an image thereof represented on a screen.

According to preferred embodiments, the sound model may isomorphically map, for at least one of the one or more data features, and ordered value range of the at least one of the one or more data features to an ordered value range of at least one of the one or more acoustic properties of the sound output. For example, if the sound model maps temperature values comprised in the data points to pitch of the sound output, the temperature range covered by said temperature values may be isomorphically mapped to the pitch range of the sound output, such that increasing temperatures may be mapped to increasing pitch and decreasing temperatures may be mapped to decreasing pitch or the other way around. By being based on an isomorphism for at least one of the one or more data features, the sound model may be able to preserve the structure of the dataset and to reflect it accordingly in the sound output.

According to preferred embodiments, the sound model may associate, based on at least one of the one or more data features, data points in said part of the dataset to one or more predefined categories, and may map each of said predefined categories to corresponding predefined values of at least one of the one or more acoustic properties of the sound output. For example, referring to the previous example in which the dataset may represent a medical image of a tissue comprising tumorous tissue and non-tumorous tissue, the sound model may map the segmentation class "tumorous tissue", as a first predefined category, to a pitch with a predefined value of 261.63 Hz (a C musical note) and the segmentation class "non-tumorous tissue", as a second predefined category, to a pitch with a predefined value of 440 Hz (an A musical note). Thereby, the generated sound output allows easily identifying whether the interaction is affecting data points belonging to the category "tumorous tissue" or "non-tumorous tissue", for example while exploring the dataset with an interaction comprising moving a cursor across a graphical representation of the dataset on a screen.

In preferred embodiments, obtaining said interaction may comprise determining at least one feature of the interaction, possibly two or more features of the interaction. Using the sound model to generate the sound output may then be further based on said at least one feature of the interaction, wherein the sound model may map said one or more data features to said one or more acoustic properties of the sound output as a function of said at least one feature of the interaction. Said at least one feature of the interaction may comprise one or more of a direction, a speed, an amplitude, a force, and a position in space. For example, if interaction is a simulated mechanical interaction with a physical system represented by the dataset, the at least one feature of the interaction may correspond to a force and a direction of the mechanical interaction, and the sound model may map the data features of the data points affected by the interaction to the one or more acoustic properties, such that, different sound outputs may be produced, for the same data points, depending on a force and a direction of the simulated mechanical interaction.

According to preferred embodiments of the invention, the sound model may be a physical model, in particular a vibrational model. A "physical model" may refer herein to any model representation of a physical system and its dynamics. The sound model may then map the at least one of the one or more data features to one or more physical parameters of the physical model, such as masses, couplings, velocities and the like. The one or more acoustic properties of the sound output may then be obtained based on vibrations of the physical model caused by the interaction. The one or more acoustic properties of the sound output may be obtained based on vibrations of the physical model caused by the interaction as a function of the previously mentioned one or more features of the interaction.

The physical model may be a simulated vibrational model which, when receiving the interaction, sets into a vibrating regime in which one or more modelled masses or objects vibrate under conditions determined by the physical parameters of the model and by the interaction, for example by the previously mentioned at least one feature of the interaction. Such vibrations can be translated into acoustic properties by means well known to the skilled person. For example, amplitude of the vibration can be directly related to loudness of the sound output and vibrating frequency of the vibration can be directly related to pitch of the sound output.

The vibrations of the vibrational model can be translated into acoustic properties of the sound output by extracting cumulative signals resulting from the vibrations of all masses within data points in a region of interest. This can be accomplished by collecting vibrations of one or more masses corresponding to the data points in the region of interest as a plurality of vibration signals and by combining the vibration signals into a composite signal, for example as a sum of the individual vibration signals. The individual vibration signals may for example be sine wave signals. The vibration signals can be combined into the composite signal using a sound synthesis method known as additive synthesis, for example as a Fourier series or as an inverse Fourier transform, to cite some implementation examples. Thereby, the vibrations of each mass contribute to the final sound profile, i.e., to the shape of the resulting sound wave, even though the individual contributions may be moderate per se.

The one or more physical parameters of the physical model may for example comprise a topology, a geometry, a number of masses, a spatial distribution of said masses, values of said masses and couplings between said masses. For example, a data set may be modelled as a cubical grid of unitary masses, with each mass representing one data point of the data set, for example one voxel, and with neighboring masses being mutually coupled by a spring-like coupling having a spring constant corresponding to an average of a value of one physical property represented by the data set, for example a density, over the two mutually coupled data points.

For instance, for a physical model including a mass parameter for data set corresponding to a medical image, the mass parameter corresponding to a given data point or voxel may take a value proportional to a value of a data feature associated to said data point or voxel, for example to a value of an intensity or a density associated to said data pint or voxel. In such cases, a mass modelled by the mass parameter of the model tends to vibrate or oscillate at a frequency inversely proportional to said value of the data feature, e.g., of said intensity or density value. Accordingly, if different mass values are used to model voxels in a region of interest in a medical image with varying values of the data feature, e.g., of said intensity or density value, different vibration frequencies will result. The resulting frequency distribution may be specific of the tissue being analyzed and may be specific of a given tissue type, for example, of tumorous or non-tumorous tissue.

The physics of the physical model corresponding to the sound model is hence correlated, via the data features of the data set, to the physics of a physical system represented by the data set. As a result, the acoustic properties of the sound output encode information about the physical properties of the physical system represented by the data set, which can be explored by interacting with the physical model. The sound model may thereby maintain the spatial and geometrical characteristics encoded in the data set and the temporal flow of the interactions.

As previously mentioned, the data set may simultaneously correspond to more than one images of one physical system. For example, the data points or voxels of the data set may comprise values corresponding to physical properties extracted from a CT scan of a tissue and may further comprise values corresponding to physical properties extracted from an MRI image of the same tissue. In these cases, some of the physical parameters of the physical model may be determined by data features corresponding to a first image, for example to a CT scan of a tissue, while some other of the physical parameters of the physical model may be determined by data features corresponding to a second image of the same physical system, for example to an MRI image of the same tissue.

According to preferred embodiments, obtaining the interaction may comprise detecting one or more of the data points receiving the interaction, wherein said at least one or more data features mapped by the sound model may correspond to the one or more data points receiving the interaction. According to this configuration, the data points contributing to the sound output are the data points that receive the interaction. For example, if interaction consists in exploring a dataset graphically represented on a screen with a cursor driven by a mouse, the at least one or more data features mapped by the sound model to acoustic features of the sound output may be data features of the data points crossed by the cursor, while other data points not crossed by the cursor may be left out of consideration and may hence not contribute to the generation of the sound output.

However, in related embodiments, the at least one or more data features mapped by the sound model may correspond to the one or more data points receiving the interaction and to other data points associated thereto. This way, the sound output may capture indirect effects of the interaction upon data points of the dataset that do not directly received interaction, but which may still react to the effect of the interaction, for example due to a coupling with the data points that do directly receive interaction. For example, if the dataset is modelled as a physical vibrational model with a plurality of masses, each mass corresponding to one data point, mutually coupled with couplings that are determined based on intensity values of the corresponding data points, for example of a given number of neighbouring data points, and interaction is simulated as a mechanical interaction upon the physical system represented by the dataset with a given force and a given direction, the data points or masses directly receiving the mechanical interaction may set into a vibrational mode that also affects other surrounding data points or masses that are indirectly coupled thereto. The vibrations of these surrounding data points or masses may also be taken into account for generating the sound output. This way, non-localized physics of the physical system represented by the dataset can also be encoded in the sound output.

According to preferred embodiments, the sound model may comprise a machine learning algorithm trained to map the one or more data features to the one or more acoustic properties of the sound output. The machine learning algorithm may be trained using a plurality of training datasets associated to corresponding ground truth values of the acoustic properties, for example via supervised learning. Using such training data, the machine learning algorithm may develop the ability to read data features of a data set and to map them to corresponding acoustic properties of a sound output to be generated.

According to preferred embodiments, the method may further comprise associating one or more predefined acoustic properties to one or more preselected data points of the data set. For example, a series of data points corresponding to voxels of a medical image corresponding to a trajectory to be followed during a surgical operation may be preselected and associated to a predetermined pitch, a predetermined loudness and a predetermined color uniquely identifying the data points corresponding to said trajectory. This allows implementing trajectory planning through the dataset. The one or more preselected data points may be preselected before implementing an operation upon a physical system represented by the dataset and the sound output can then be used to track whether a subsequent interaction with the dataset corresponds to the preselected data points, for example to a predetermined trajectory.

As previously mentioned, the data points may be obtained by one or more sensor devices, for example by one or more sensor devices of a medical imaging device. In such cases, the sound output can be generated based on the data set obtained by the one or more sensor devices, preferably in real time. For example, the data set may correspond to a CT scan of a body of a patient obtained in real time, for instance during surgical operation on the body of the patient.

The method according to the first aspect of the invention may be used, according to a second aspect of the invention, as part of a method of validating labels assigned to ground truth input data. For this use, the method according to the first aspect of the invention may be used for generating a sound output from an interaction with the ground truth input data set. Correctly assigning ground truth values to data is an essential task in many scientific and technological fields involving the use of data science. Traditionally, human-assigned labels are validated by comparing the labels with original data by visual-based approaches. For example, labels assigned to medical images, like a CT scan or an MRI image, may be validated by visual side-by-side comparison to an original image. This known technique is prone to errors and misinterpretation. By using the method according to the first aspect of the invention for validating labels assigned to ground truth input data, labels can be validated relying on the sound output generated by the method such that a user or validator may be able to simultaneously view the original image while listening to the sound output, thereby achieving high validation accuracy and efficiency and avoiding the errors related to visual validation.

The method according to the first aspect of the invention may further be used, according to a third aspect of the invention, as part of a method of training a machine learning algorithm. For this use, the sound output generated by the method according to the first aspect of the invention may be used as part of training input data for training the machine learning algorithm. The auditory information encoded in the sound output generated by the method according to the first aspect of the invention can be treated, in particular along with other data, to enhance and augment the training effect. For example, a machine learning algorithm to be trained based on medical images and/or patient information may further be trained using the sound output as generated by the method according to the first aspect of the invention for datasets representing said medical images as part of the training data. This provides additional information and context to the machine learning algorithm, which can improve the accuracy and performance of the algorithm.

A further use of the method according to the first aspect of the invention may be part of a method of applying a surgical treatment or a diagnostic treatment to a patient, wherein the method according to the first aspect of the invention may be used for generating a sound output based on an interaction with a data set corresponding to a representation of a body part of the patient.

A fourth aspect of the invention refers to a computer program comprising executable instructions which, when executed by a processor, cause the processor to implement a method according to any of the preceding aspects of the invention.

A fifth aspect of the invention refers to a system for generating a sound output based on an interaction with a data set. The system comprises an interaction module and a sound module. The interaction module is configured for obtaining an interaction with at least a part of the data set. The sound model module is configured for generating a sound output based on said interaction according to the method of the first aspect of the invention.

The interaction module and the sound module may each correspond to a processing unit, which may be hardware-based and/or software-based. Alternatively, the interaction module and the sound module may correspond to exactly one processing unit, which may be hardware-based and/or software-based.

Preferably, the system may further comprise a sound generator for generating an audible sound signal according to the sound output generated by the sound model module. The sound generator may for example comprise an amplifier and/or at least one loudspeaker.

In preferred embodiments, the system may further comprise a user input device configured for allowing a user to input the interaction to the interaction module. Preferably, the user input device may comprise at least one of a touch pad, a touch screen, a keyboard, a mouse, a joystick, a pedal, a gaze, a camera, and a tracker.

BRIEF DESCRIPTION OF THE DRAWINGS

Fig. 1 shows a schematic illustration of a system according to the fifth aspect of the invention.

Fig. 2 is a flow diagram of a method according to the first aspect of the invention, implemented by the system of Fig. 1.

Fig. 3 is a schematic representation of a data set.

Fig. 4 shows a schematic representation of an interaction with the data set of Fig. 3 in the form of a user-determined trajectory.

Fig. 5 shows a schematic representation of a physical model than can be used as sound model by the system of Fig. 1 for the data set of Fig. 3.

Fig. 6 illustrates an experimental example of the invention.

DETAILED DESCRIPTION OF PREFERRED EMBODIMENTS

For the purposes of promoting an understanding of the principles of the invention, reference will now be made to a preferred embodiment illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to one skilled in the art to which the invention relates.

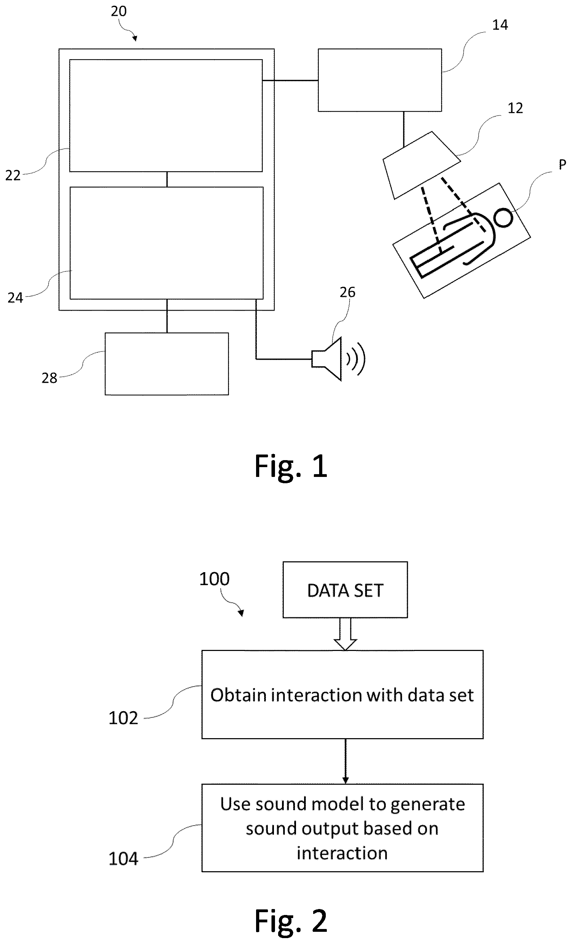

Fig. 1 illustrates a system 20 according to the fifth aspect of the invention, which is configured for implementing a method 100 for generating a sound output based on an interaction with a data set according to the first aspect of the invention. Fig. 2 shows a flow diagram of the method 100 implementable by the system 20. The system 20 may be a hardware-based system, for example implemented as an independent processor or CPU, or may be a software-based system executable by a processor.

As represented in Fig. 1, a body part of a patient P is being scanned by a sensor device 12, which can for example correspond to a medical imaging device such as CT scanner or an MRI scanner or the like. The sensor device 12 scans a part of the body of the patient P and codifies the scan results as data that is processed into a resulting data set by the CPU 14, which is connected to the system 20. The CPU 14 may be a CPU of the medical imaging device, an external an independent CPU or a CPU of the system 20.

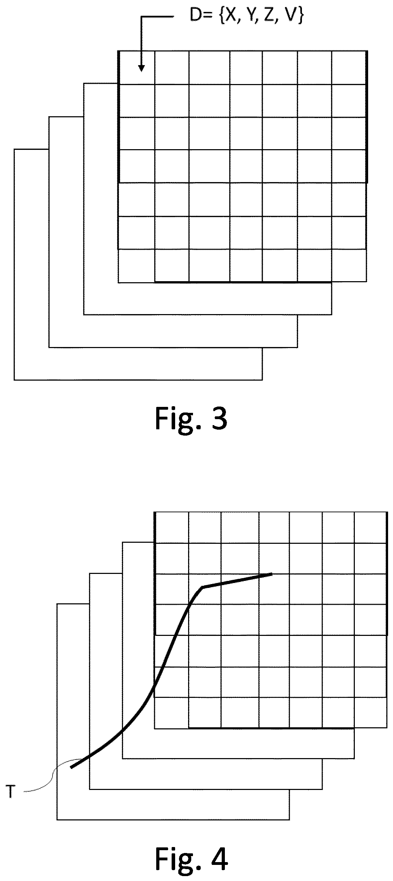

The system 20 receives the data set from the CPU 14. An example of the data structure of the data set is represented in Fig. 3. As seen in Fig. 3, the multi-dimensional data set comprises a plurality of data points arranged in a 3D grid structure, so that each of the data points can be associated to a position in space by three data features corresponding to three spatial coordinates, e.g., X, Y and Z. In the simplistic example shown in Fig. 3, a plurality of 2D tables of data points is shown, wherein each of the 2D tables may exemplarily correspond to an XY plane and different tables may correspond to different values of the Z coordinate. Each of the data points hence corresponds to a voxel of the medical image provided by the sensor 12 or to a subregion thereof.

Further, each of the data points can comprise one or more additional data features, for example a density value measured by the CT scanner 12 at a corresponding spatial region of the body of the patient P. Thus, each data point D may be represented by a vector comprising four values of respective data features: a value for a spatial X coordinate, a value for a spatial Y coordinate, a value for a spatial Z coordinate, and a density value V. Notably, this is just an example and other combinations of data features, in particular any of a spatial feature, a time feature, a physical property, and/or a data label, and/or a different number of data features per data point can be used in other related examples.

The system 20 receives the data set from the CPU 14. The system 20 comprises an interaction module 22, a sound model module 24, a sound generator 26 and a user input device 28.

The interaction module 22 is configured for obtaining, at step 102 of the method 100 shown in Fig. 2, an interaction with at least a part of the dataset. An example of such interaction is illustrated in Fig. 4 for the dataset of Fig. 3. In this example, the interaction corresponds to a trajectory through a graphical representation of the dataset determined by a user. For example, the user input device 28 can comprise a mouse and the user can determine the trajectory T illustrated in Fig. 4 through the graphical representation of the dataset illustrated in Fig. 3 by correspondingly moving the mouse and thereby guiding the cursor on a screen representing the graphical representation of the dataset, e.g., a graphical representation of the CT scan obtained by sensor device 12. The interaction module determines positions in 3D space along the trajectory T, i.e., three spatial features of the trajectory (e.g., an X coordinate, a Y coordinate and a Z coordinate).

The sound model module 24 is configured for generating, at step 104 of the method 100 shown in Fig. 2, a sound output based on the interaction, i.e., in this case based on the user-determined trajectory T. The sound model maps one or more of the data features of the data points in the dataset represented in Fig. 3 to one or more acoustic properties of the sound output. For example, the sound model can isomorphically map the density values V of the data points crossed by the user-determined trajectory T to a pitch of the sound output and can output the sound output having pitch values isomorphically mapped to the density values V of the data points.

The sound output outputted can be transmitted to the sound generator 26, which can comprise an amplifier and/or a loudspeaker, to generate an audible signal based on the sound output having a corresponding pitch. A person listening to such audible signal based on the sound output hence obtains, via auditory perception, information about the density measured by the CT scan 12 for regions of the body of the patient P represented by the data points crossed by the user-determined trajectory T.

The user-determined trajectory T may for example correspond to an intended trajectory to be followed by a surgical instrument during a surgical operation, in which case a surgeon may memorize the pitch sequence of the sound output corresponding to the trajectory T in order to use the system 20 in real-time during the surgical operation to monitor whether the predetermined trajectory is being correctly followed.

The data points can comprise values of additional data features, for example a data label corresponding to a segmentation class indicating, for each data point or voxel, whether the data point corresponds to muscular tissue or to bone tissue. The sound model may then map each of such additional data features to acoustic properties of the sound output. For example, the sound model may be configured to generate the sound output as a continuous signal having the corresponding pitch when the trajectory is crossing data points having a data label indicating that the data point corresponds to the segmentation class "muscular tissue" and as a pulsing or intermittent signal having the corresponding pitch when the trajectory is crossing data points having a data label indicating that the data point corresponds to the segmentation class "bone tissue".

Additionally or alternatively, the sound model can apply the isomorphic map between density and pitch in a first frequency range, for example 16 Hz to 28 Hz, when the trajectory is crossing data points having a data label indicating that the data point corresponds to the segmentation class "muscular tissue" and in a second frequency range different from the first frequency range, for example 260Hz to 494 Hz, when the trajectory is crossing data points having a data label indicating that the data point corresponds to the segmentation class "bone tissue".

If the data points comprise an additional data feature used for indicating whether a data point corresponds to a predefined category, for example "tumorous tissue", the sound model can be configured to map said additional data feature, when having a value corresponding to "tumorous tissue", to a predetermined loudness of the sound output allowing a user to clearly identify a sudden increase or decrease in loudness audibly marking that the trajectory T is now crossing a data point identified as "tumorous tissue".

Alternatively, the predetermined loudness may be indicative of one or more preselected data points, for example of one or more data points preselected by a surgeon as corresponding to an incision point, which may for example help a surgeon identify, via auditory perception, a correct incision point preselected on a screen.

Rather of using an isomorphism mapping a density range to a pitch range, the sound model can additionally or alternatively comprise a machine learning algorithm trained to infer a pitch value from a density value. The machine learning algorithm can be for example trained as explained above, based on a plurality of data sets and trajectories with sound outputs associated thereto via supervised learning.

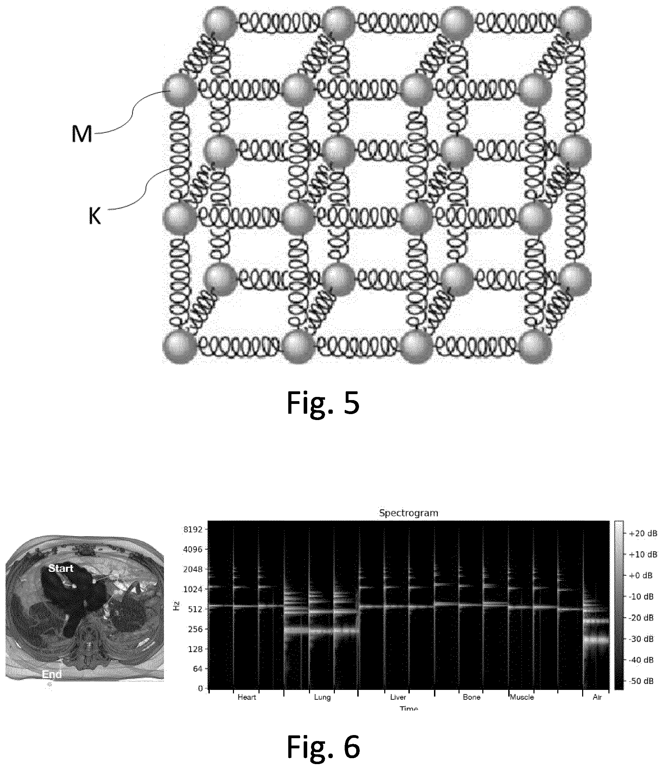

The sound model module 24 can be configured to use a sound model corresponding to a physical model. One simplistic example of such a model is the vibrational model represented in Fig. 5 for the data set represented in Fig. 3. According to this model, the position in space of each of the data points, e.g., each voxel of the CT scan, is represented by a vibrating mass M, which in this case results in a regular cubic structure of masses M, which in a simplistic approximation may be identical masses M. Notably, other physical models with other topologies, not necessarily a regular cubic structure, can be used. The masses M are coupled to immediate neighbors in the cubical grid by a spring-like linear coupling based on Hook's law with a value of the stiffness constant K being determined by the averaged values of density averaged over each two neighboring data points (masses M).

The body of the patient P represented in Fig. 1, as an example of an arbitrary physical system, is then encoded in the data set as represented by the physical model illustrated in Fig. 5. The dynamics of the physical model are then representative of the physical properties of the body of the patient P and of its dynamic evolution. A mechanical action upon the body of the patient P, for example during a surgical operation, can be represented or simulated as an interaction captured by the interaction module 22 having a plurality of interaction features, for example a direction, a location of space and a force. The sound model module 24 can be configured to compute the vibrational effects of an interaction having such interaction features upon the physical mode, in this case upon the grid of mutually spring-coupled vibrating masses M acting as coupled harmonic oscillators. The interaction will excite the masses M of the physical model and set them into vibration and the vibrations of the masses M can be registered as individual vibration signals combining into the sound output.

In some related embodiments, the masses M can take values proportional to an intensity value associated to said data point or voxel, such that the masses M will vibrate at a frequency inversely proportional to said intensity value. Accordingly, for voxels in a region of interest of the CT scan with varying intensity values, different vibration frequencies will result. A specific frequency distribution will in turn result in a sound profile (e.g., a chord-like frequency distribution) representing the underlying data structures. For example, when interacting with a physical model or a portion of a model representing bone structures, the resulting sound encoded in the sound output may resemble that of hitting a bone with a stab, whereas when interacting with a physical model or a portion of a model representing soft tissue structures, the resulting sound encoded in the sound output may be reminiscent of the sound of cutting or hitting meat.

The vibrations of each of the masses M can be extracted as corresponding vibration signals, for example in the form of sine waves, and a cumulative signal can be obtained by the sound model module 24 for all voxels or a subset thereof, for example for all voxels in a region of interest of the CT scan, as a cumulative signal corresponding to the inverse Fourier transform of all individual vibration signals. The cumulative signal can correspond to the sound output, which encodes, in the form of acoustic properties thereof, the physics of the underlying vibrational model. The sound model module 24 can transmit the sound output comprising the cumulative signal to the sound generator 26, so that the sound generator 26 can emit a sound based thereon.

Notably, the interaction also shapes the resulting sound. For example, the proximity or distance of each vibrating mass from a location of the interaction plays a significant role in shaping the resulting sound output.

EXAMPLES

The mass-interaction physics methodology (cf. Villeneuve, J. and Leonard, J., 2019. Mass-interaction physical models for sound and multi-sensory creation: starting anew. In Proceedings of the 16th Sound & Music Computing Conference (pp. 187-194)) allows the formulation of physical systems, such as the linear harmonic oscillator, which comprise two fundamental constituents: masses, representing material points within a 3D space, with corresponding inertial behaviors, and connecting springs, signifying specific types of physical couplings such as viscoelastic and collision between two mass elements.

To represent and compute discretized modular mass-interaction systems, a widely used method involves applying a second-order central difference scheme to Newton's second law, which states that force F is equal to mass $m$ times acceleration $a$, or the second derivative of its position vector $x$ with respect to time $t$.

The total force exerted by the dampened spring, denoted as $F(t_n) = F_S(t_n) + F_D(t_n)$ at a time $t_n$, where Fs represents the elastic force exerted by a linear spring (the interaction) with stiffness K, connecting two masses $M_1$, $M_2$ located at positions $x_1$, $x_2$, can be expressed using the discrete-time equivalent of Hooke's law. Similarly, the friction force $F_D$ applied by a linear damper with damping parameter $z$ can be derived using the Backward Euler difference scheme with the discrete-time inertial parameter $Z = z/\Delta T$. $F(t_n)$, which is a vector quantity, is applied symmetrically to each mass in accordance with Newton's third law: $F_{2 \to 1}(t_n) = - F(t_n)$ and $F_{1 \to 2}(t_n) = + F(t_n)$. The combination of forces applied to masses and the connecting spring yields a linear harmonic oscillator as described in

$$X(t_{n+1}) = \left(2 - \frac{K+Z}{M}\right)X(t_n) + \left(\frac{Z}{M} - 1\right)X(t_{n-1}) + \frac{F(t_n)}{M}$$

which is a fundamental type of the mass-interaction system. This system is achieved by connecting a dampened spring between a mass and a fixed point $x_1(t_n) = 0$.

A mass-interaction system can be extended to a physical model network with an arbitrary topology by connecting the masses via dampened springs. The connections are formalized as a routing matrix T of dimensions $r \times c$, where $r$ denotes the number of mass points in the physical model network, and $c$ represents the number of connecting springs, each having only two connections. A single mass can be connected to multiple springs in the network.

An interaction module excites the model by applying a force to one or more input masses of the model. A function maps the intensities of the input data to $M, K, Z,$ and F. Therefore, the input force is propagated through the network according to the above equation and observed by the output masses.

The output masses are hence affected by the oscillation of all masses activated in the model with various frequencies and corresponding amplitudes, resulting in a specific sound profile, i.e., tone color. This tone color represents the spatial structure and physical properties of the physical system represented by the data set, which is transformed into the features of the output sound.

Experiment and results

Studies were carried out to evaluate the feasibility of the method of the first aspect of the invention for creating a model that is capable of generating discernible sound profiles in accordance with underlying anatomical structures. In particular, the aim was to differentiate between sounds of a set of tissue types. Through empirical experimentation on an abdominal CT volume (cf. Luo, X., Liao, W., Xiao, J., Chen, J., Song, T., Zhang, X., Li, K., Metaxas, D.N., Wang, G. and Zhang, S., 2022. WORD: A large scale dataset, benchmark and clinical applicable study for abdominal organ segmentation from CT image. Medical Image Analysis, 82, p.102642.), the inventors determined a model configuration that achieves stability and reduces noise to the desired level. The shape of the topology was a 3D cube of size representing 7 mm $\times$ 7 mm $\times$ 7 mm of the CT volume. The inter-mass connections were established at the grid spacing distance of 1 $mm$ between each mass and its adjacent neighbor masses. All the masses located on the surface of the model were set as fixed points. To excite the model, equal forces were applied to the center of the model in a 3D direction and observed at the same position. A region of interest (RoI) was obtained by selecting 3D cubes with the same topology and size in the CT volume. The intensities in the RoI were transformed to define the model parameters. The spring parameters $K$ and $Z$ were derived by averaging the intensities of their adjacent CT voxels, by a linear mapping and $M$ and F were set to constant values. The inventors defined a sequence of RoI starting in the heart, passing through lung, liver, bone, muscle, and ending in air in a 16-steps trajectory, as shown in Figure 6. For visualizing the trajectory and processing image intensities, the inventors used ImFusion Suite (see Zettinig, O., Salehi, M., Prevost, R. and Wein, W., 2018. Recent Advances in Point-of-Care Ultrasound Using the ImFusion Suite ImFusion Suite for Real-Time Image Analysis. In Simulation, Image Processing, and Ultrasound Systems for As-sisted Diagnosis and Navigation: International Workshops, POCUS 2018, BIVPCS 2018, CuRIOUS 2018, and CPM 2018, Held in Conjunction with MICCAI 2018, Granada, Spain, September 16-20, 2018, Proceedings (pp. 47-55). Springer International Publishing). For generating physical models and generating sound, the inventors used mass-interaction physical modelling library (Mass Interaction Physics in Java/Processing Homepage, https://github.com/mi-creative/miPhysics\_Processing) for the Processing sketching environment (see https://processing.org/). Visual demonstrations of the models, along with the corresponding sound samples, are provided in the supplementary material, along with additional explanations.

A mel spectrogram is a visual representation of the frequency content of an audio signal, where the frequencies are mapped to a mel scale, which is a perceptual frequency scale based on how humans hear sounds. Therefore, the inventors used this representation to show the frequency content of the resulting sound of the trajectory, presented in Figure 6.

In Figure 6, the spectrogram on the right illustrates the sound profiles of the tissues corresponding to the sequence of RoIs marked as yellow on the abdominal CT (left). The green spheres highlight the excitation points.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

**Claims**

1. A method (100) for generating a sound output based on an interaction with a data set, wherein the data set comprises a plurality of data points, each data point storing one or more data features, wherein the method comprises:

   obtaining (102) an interaction with at least a part of the data points of the data set,
   using (104) a sound model to generate a sound output based on said interaction, wherein
   the sound model maps at least one of the one or more data features to one or more acoustic properties of the sound output as a function of the interaction,

   wherein at least one of the one or more data features corresponds to one of a spatial feature, a time feature, a physical property, and a data label; and
   wherein at least one of the one or more acoustic properties of the sound output corresponds to one of pitch, pulsing frequency, duty cycle, loudness, and tone colour;

   wherein the sound output is generated having said one or more acoustic properties.

2. The method of claim 1, wherein, the sound model isomorphically maps, for at least one of the one or more data features, an ordered value range of the at least one of the one or more data features to an ordered value range of at least one of the one or more acoustic properties of the sound output.

3. The method of claim 1, wherein, the sound model associates, based on at least one of the one or more data features, data points in said part of the data set to one or more predefined categories, and maps each of said predefined categories to corresponding predefined values of at least one of the one or more acoustic properties of the sound output.

4. The method of any of the preceding claims, wherein obtaining said interaction comprises determining at least one feature of the interaction, wherein using the sound model to generate the sound output is further based on said at least one feature of the interaction, wherein the sound model maps said one or more data features to said one or more acoustic properties of the sound output as a function of said at least one feature of the interaction, wherein said at least one feature of the interaction preferably comprises one or more of a direction, a speed, an amplitude, a force, and a position in space.

5. The method of any of the preceding claims, wherein the sound model is a physical model, in particular a vibrational model, wherein the sound model maps the at least one of the one or more data features to one or more physical parameters of the physical model, and wherein the one or more acoustic properties of the sound output are obtained based on vibrations of the physical model caused by the interaction.

6. The method of claims 4 and 5, wherein the one or more acoustic properties of the sound output are obtained based on vibrations of the physical model caused by the interaction as a function of the one or more features of the interaction.

7. The method of any of the preceding claims, wherein obtaining the interaction comprises detecting one or more of the data points receiving the interaction, wherein said at least one or more data features mapped by the sound model correspond to the one or more data points receiving the interaction, wherein said at least one or more data features mapped by the sound model preferably correspond to the one or more data points receiving the interaction and to other data points associated thereto.

8. The method of any of the preceding claims, wherein the sound model comprises a machine learning algorithm trained, using a plurality of training data sets, to map the one or more data features to the one or more acoustic properties of the sound output.

9. The method of any of the preceding claims, wherein the data set corresponds to one or more biomedical images, preferably one or more of a CT image, an MRI image, a PET image, a SPECT image and an OCT image.

10. The method of any of the preceding claims, wherein the method further comprises associating one or more predefined acoustic properties to one or more preselected data points of the data set.

11. The method of any of the preceding claims, wherein the data set is obtained from one or more sensors, and wherein the sound output is generated based on the data set obtained by the one or more sensors, preferably in real time.

12. A method of validating labels assigned to ground truth input data, wherein the method comprises using the method of any of the preceding claims for generating a sound output from an interaction with the ground truth input data set.

13. A method of training a machine learning algorithm comprising using a sound output generated according to the method of any of claims 1-11 as part of training input data for training the machine learning algorithm.

14. A computer program comprising executable instructions which, when executed by a processor, cause the processor to implement the method of any of the preceding claims.

15. A system (20) for generating a sound output based on an interaction with a data set, wherein the system comprises:

a interaction module (22) configured for obtaining a interaction with at least a part of the data set, and
a sound model module (24) configured for generating a sound output based the interaction according to the method defined in any of claims 1 to 13;
wherein the system preferably further comprises a sound generator (26) for generating an audible sound signal according to the sound output generated by the sound model module (24), preferably further comprising a user input device (28) configured for allowing a user to input the interaction to the interaction module (22), wherein the user input device more preferably comprises at least one of a touch pad, a touch screen, a keyboard, a mouse, a joystick, a pedal, a gaze, a camera, and a tracker.

# Fig. 1

DATA SET

Obtain interaction with data set

Use sound model to generate sound output based on interaction

# Fig. 2

Fig. 3

Fig. 4

M

K

## Fig. 5

## Fig. 6

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/231249 A1 (DASCALU AVI [IL]) 1 August 2019 (2019-08-01) * figures 1-3,7 * * paragraph [0110] - paragraph [0148] * | 1-15 | INV. G06F3/16 G06N20/00 A61B5/00 |
| X | MATINFAR SASAN ET AL: "From Tissue to Sound: Model-Based Sonification of Medical Imaging", 1 October 2023 (2023-10-01), MEDICAL IMAGE COMPUTING AND COMPUTER ASSISTED INTERVENTION - MICCAI 2023; [LECTURE NOTES IN COMPUTER SCIENCE; LECT.NOTES COMPUTER], SPRINGER NATURE SWITZERLAND, CHAM, PAGE(S) 207 - 216, XP047671656, ISSN: 0302-9743 ISBN: 978-3-031-43995-7 [retrieved on 2023-10-01] * the whole document * | 1-15 | |
| X | US 2019/336063 A1 (DASCALU AVI [IL]) 7 November 2019 (2019-11-07) * figures 6,7 * * paragraph [0033] - paragraph [0084] * | 1-15 | TECHNICAL FIELDS SEARCHED (IPC) G06F G06N A61B |

The present search report has been drawn up for all claims

| Place of search | Date of completion of the search | Examiner |
|---|---|---|
| The Hague | 27 February 2024 | Alliot, Sylvain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 535 160 A1**

## ANNEX TO THE EUROPEAN SEARCH REPORT
## ON EUROPEAN PATENT APPLICATION NO.

EP 23 20 2202

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2024

| Patent document cited in search report | Publication date | Patent family member(s) | Publication date |
|---|---|---|---|
| US 2019231249 A1 | 01-08-2019 | EP 3478160 A1 | 08-05-2019 |
| | | US 2019231249 A1 | 01-08-2019 |
| | | WO 2018005316 A1 | 04-01-2018 |
| US 2019336063 A1 | 07-11-2019 | NONE | |

EPO FORM P0459

For more details about this annex : see Official Journal of the European Patent Office, No. 12/82

17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VILLENEUVE, J** ; **LEONARD, J.** Mass-interaction physical models for sound and multi-sensory creation: starting anew. *In Proceedings of the 16th Sound & Music Computing Conference*, 2019, 187-194
- **LUO, X** ; **LIAO, W.** ; **XIAO, J.** ; **CHEN, J.** ; **SONG, T.** ; **ZHANG, X** ; **LI, K.** ; **METAXAS, D.N.** ; **WANG, G** ; **ZHANG, S.** WORD: A large scale dataset, benchmark and clinical applicable study for abdominal organ segmentation from CT image. *Medical Image Analysis*, 2022, vol. 82, 102642

- Recent Advances in Point-of-Care Ultrasound Using the ImFusion Suite ImFusion Suite for Real-Time Image Analysis. **ZETTINIG, O.** ; **SALEHI, M.** ; **PREVOST, R** ; **WEIN, W**. In Simulation, Image Processing, and Ultrasound Systems for As-sisted Diagnosis and Navigation: International Workshops, POCUS 2018, BIVPCS 2018, CuRIOUS 2018, and CPM 2018, Held in Conjunction with MICCAI 2018, Granada, Spain. Springer International Publishing, 2018, 47-55